# EUROPEAN PATENT SPECIFICATION

(11) **EP 3 537 893 B1**
(45) Date of publication and mention of the grant of the patent: **15.07.2020**
(21) Application number: 17808027.1
(22) Date of filing: 17.11.2017
(51) Int. Cl.: A23L 27/10, A23L 27/40, A23L 33/105, A23L 33/155, C12N 1/12

(54) **METHOD FOR PRODUCING A CULINARY CONDIMENT WITH DUNALIELLA SALINA AND SEA SALT**
VERFAHREN ZUR HERSTELLUNG EINES SPEISENGEWÜRZES MIT DUNALIELLA SALINA UND MEERSALZ
PROCÉDÉ DE PRODUCTION D'UN CONDIMENT CULINAIRE AVEC DUNALIELLA SALINA ET DU SEL MARIN

(30) Priority: 18.11.2016 ES 201631488
(43) Date of publication of application: 18.09.2019
(73) Proprietor: Instituto Tecnologico de Canarias, S.A. (ITC), 35003 Las Palmas (ES)
(72) Inventor: PORTILLO HAHNAFELD, Eduardo, 35003 Las Palmas (ES); SUÁREZ VEGA, Antonio, 35003 Las Palmas (ES); MENDOZA GUZMÁN, Héctor, 35003 Las Palmas (ES); DE LA JARA VALIDO, Adelina, 35003 Las Palmas (ES); FREIJANES PRESMANES, Karen, 35003 Las Palmas (ES); CLEMENTE JANEIRO ASSUNÇÃO, Patricia Alexandra, 35003 Las Palmas (ES); RODRIGUEZ ESTUPIÑAN, David, 35003 Las Palmas (ES)
(74) Representative: Carvajal y Urquijo, Isabel
(86) International application number: PCT/EP2017/079540
(87) International publication number: WO 2018/091628

(56) References cited:
- WO-A2-2012/153955
- UA-C2- 85 336
- MARIO GIORDANO ET AL: "Dunaliella salina (Chlorophyceae) Affects the Quality of NaCl Crystals", CRYPTOGAMIE. ALGOLOGIE, vol. 35, no. 3, 1 August 2014 (2014-08-01) , pages 285-302, XP055440782, FR ISSN: 0181-1568, DOI: 10.7872/crya.v35.iss3.2014.285
- Anonymous: "Dunaliella - Wikipedia", Wikipedia, 26 May 2016 (2016-05-26), pages 1-3, XP055441501, Retrieved from the Internet: URL:https://en.wikipedia.org/w/index.php?t itle=Dunaliella&oldid=722190097 [retrieved on 2018-01-17]

## Description

### Object of the invention

The present invention relates to a method for producing a culinary condiment with Dunaliella salina and sea salt which has a high content of β carotenes.

The present object belongs to the sector of the food industry, specifically that which is directed at producing culinary condiments and additives which are used in the preparation and enrichment of food products.

### Background of the invention

A condiment is normally required in culinary preparations which boosts or enhances the intrinsic flavor of the food during its consumption. For example, sea salt, salt/pepper mixture, salt, parsley and garlic mixture, dehydrated algae, amongst other mixtures of condiments. It is important for this condiment to be incorporated into the food product in the correct and suitable dose from the point of view of the health of individuals and from an organoleptic point of view.

At present in the food industry, there is a need for new culinary condiments or mixtures of condiments which, following their incorporation and mixing, provide the food preparations with greater nutritional value. One example of the foregoing is the use of algae, microalgae and similar, as bioactive ingredients when producing food products.

It is known that microalgae constitute a natural reserve for the development and design of new foods. For example, it is known that Dunaliella salina is a microalgae which intracellularly synthesizes and accumulates large quantities of β carotene isomers in a hyper saline medium (halophilic microorganism) with high exposure to sun light and at high temperatures.

The microalgae *Dunaliella salina,* in addition to being known for its very high tolerance to hypersaline media, has significant antioxidant activity which makes it interesting for applying in the food, cosmetics and pharmaceutical industries.
The possibility to produce a *Dunaliella salina*-containing salt with durable properties appears very attractive; however, the mechanisms through which this alga can interact with the salt crystals have only been described in laboratory-scale experiments, in quite standardized and unnatural conditions (e.g. Giordano et al 2014).
One example is the document UA85336C2, which discloses a salt immunoseasoning comprising table salt, biomass of the seaweed *Dunaliella salina* as colloidal solution of the seaweed biomass, sodium ascorbate, ascorbic acid, beta-carotene, sea macro- and micronutrients, carbon dioxide and essential eucalyptus oil. The method for obtaining the salt immunoseasoning includes mixing and drying the table salt with additives with the table salt and the immunoadditive of the seaweed *Dunaliella salina* mixed in a ratio of 30:1-10:1. The mixture is dried at a temperature of 30-50 °C in an atmosphere of carbon dioxide, and after cooling essential eucalyptus oil is added.
WO2012153955A2 relates to a method for producing mineral salt by processing deep sea water, and more particularly, to a method for producing salts containing useful ingredients such as β-carotene, which are contained in algae such as dunaliella, spirulina, and chlorella. The method for producing salt containing useful algae ingredients comprises the steps of: (a) sterilizing or filtering and concentrating seawater; (b) culturing algae in the seawater concentrated in step (a); and (c) evaporating the seawater in which the algae is cultured in step (b). The invention also relates to the salt produced by the method. Using the above-described method, salt containing useful ingredients, such as β-carotene, may be produced consistently in an inexpensive manner, and said method can thus be combined with a method for effectively reusing concentrated seawater, which is by-product of sea water desalination, thereby effectively preserving coastal environments.

However, in the food industry, in the preparation and design of culinary condiments, the incorporation of Dunaliella salina is not viable in current practice. If this microalgae is dehydrated and/or lyophilized, as is done in current different processes, the β carotene ends up degrading through enzymatic action, exposure to light and to oxygen and, even more quickly, in the presence of salt due to the fact that the oxidative reactions are accelerated (less than 30 days).

The present invention is directed at resolving the problem cited above and meeting the need for a new condiment with a high content of β carotenes; in this case, the invention proposes the preparation of a condiment with Dunaliella salina, which is present in an integral and lasting manner, and sea salt.

### Description of the invention

The invention relates to a method for producing a culinary condiment by joint crystallization of salt and the microalga *Dunaliella salina* in sea salt evaporation ponds, which allows the microalgae biomass to be integrated, in integral cellular state, inside the salt, wherein the method is carried out by means of a system formed by multiple rotary paddle wheels arranged and coupled in a non-permanent manner via a rotary cylindrical bar, actuated by renewable energy, wind and solar, with continuous and slow movement of less than 10 revolutions per minute, wherein the paddles are introduced in the central part of a sea evaporation pond and partially submerged, barely a few millimeters into the surface of the hypersaline water, forming, at their edges and along the entire width of the paddles, a continuous dripping which gives rise to the joint precipitation of the salt and the microalgae biomass in live state, wherein in said sea salt evaporation pond there is naturally a high concentration of *Dunaliella salina* and the medium has reached a crystallization salinity greater than 250 g/L. Sea salt which is traditionally obtained from coastal salts is generated in the evaporation ponds together with this microalgae, coloring the salt produced with a pink tonality. The evaporation ponds are tide-pools or generally rectangular enclosures, with shallow depth in which the natural evaporation of the sea water is produced. When the sea water deposited in these evaporation ponds reaches crystallization salinity, it usually has a high density of these microalgae as well as a high concentration of β carotene. However, in the traditional process for collecting salt, by dragging shovels, fleur de sel, by surface filtration as well as washing which are carried out to preserve optimal sanitary hygienic conditions of the salt prepared, this microalgae is removed from the final product.

Alternatively, considering the processes existing at present in the prior art, the present invention develops a joint crystallization process (salt and microalgae) in the mentioned ponds, which allows the microalgae biomass to be integrated, in integral cellular state, inside the salt.

Integral cellular state is understood as the state in which the cell walls of the microalgae are maintained intact, without breaking, and, therefore, there is no outlet nor substantial modification of its intracellular content.

This process is carried out by means of a paddle system (system formed by multiple rotary paddle wheels partially submerged in water which are arranged and coupled in a non-permanent manner via a rotary cylindrical bar which are actuated by renewable energy, wind and solar) with continuous and slow movement (less than 10 revolutions per minute). The paddles are introduced barely a few millimeters into the surface of the hypersaline water and central part of the pond, forming, at its edges and along the entire width of the same, a continuous dripping which ends jointly precipitating the salt and the algae biomass in live state (incorporation of Dunaliella salina in the salt).

In this process of precipitation by continuous dripping, the microalgae, due to the effects of the crystallization, is compressed and reduced, but allows the cell integrity to be preserved, in particular the cell wall. In this way, the degradation of the β carotene is prevented since it is protected inside the microalgae as a natural organic pigment.

Therefore, using the present method, the live cell is encapsulated, remains trapped, in the salt. Thus, the method allows the cell integrity of Dunaliella salina to be maintained or safeguarded and prevents the degradation of intracellular β carotene since it is protected inside the microalgae and allows its use as a natural organic pigment.

The production method concludes with the packaging and storage of the condiment, prior to its distribution.

The method of the invention provides a culinary condiment which, obtained by means of a production method defined as described above, comprises Dunaliella salina and sea salt. Considering that the microalgae remains in integral cellular state in the salt, the present condiments provides characteristic and different color, aroma and flavor, both to the salt and to the cooked and semi-prepared foods which incorporate it, which will be maintained and preserved with the passing of time.

The condiment obtained here is generally used in the food industry and, in particular in restaurant establishments as a natural colorant due to its high content of β carotene (E160a) and as a flavoring and flavor enhancer of foods cooked and prepared therewith.

Among the advantages of the present method over the traditional processes, are the following, amongst others:
- Dunaliella salina is stably mixed in live state with sea salt, without degradation of intracellular β carotene,
- live cells of this microalgae are incorporated into the sea salt and are encapsulated, conserving their cellular integrity, and
- a new condiment based on Dunaliella salina and sea salt is obtained.

### Exemplary embodiment

In order to carry out the method of the present invention, as described above, a modular (wheels independent of each other) and mobile (transportable from one pond to another) system of paddles (system described above) is used. In this case, it is transported to the ponds of a traditional saline coast where there is naturally a high concentration of Dunaliella salina and where the medium has reached a crystallization salinity greater than 250 g/l.

In this way, (with the rotary paddle wheels and immersion of the edges of the same) the water can be stirred and all the salt from the pond with Dunaliella salina incorporated is precipitated on the surface of the paddles (step for incorporating the microalgae in the salt).

Once this reddish salt with Dunaliella salina on the paddles has been collected from the pond, the stirring system is taken away to the processing room to scrape the blades with a type of spatula system and remove the salt from the same and restart the process in another pond in optimal conditions for successively developing said process.

The sea salt with Dunaliella salina incorporated, said culinary condiment being packaged and stored for its distribution to different entities of the food industry, centers and restaurant establishments, etc.

## Claims

1. Method for producing a culinary condiment by joint crystallization of salt and the microalga *Dunaliella salina* in sea salt evaporation ponds, which allows the microalgae biomass to be integrated, in integral cellular state, inside the salt,
wherein the method is carried out by means of a system formed by multiple rotary paddle wheels arranged and coupled in a non-permanent manner via a rotary cylindrical bar, actuated by renewable energy, wind and solar, with continuous and slow movement of less than 10 revolutions per minute,
wherein the paddles are introduced in the central part of a sea evaporation pond and partially submerged, barely a few millimeters into the surface of the hypersaline water, forming, at their edges and along the entire width of the paddles, a continuous dripping which gives rise to the joint precipitation of the salt and the microalgae biomass in live state,
wherein in said sea salt evaporation pond there is naturally a high concentration of *Dunaliella salina* and the medium has reached a crystallization salinity greater than 250 g/L.

## Patentansprüche

1. Verfahren zur Herstellung eines kulinarischen Gewürzes durch gleichzeitige Kristallisation von Salz und der Mikroalge *Dunaliella salina* in Meersalzverdunstungsbecken, wodurch die Mikroalgenbiomasse in integralem Zellzustand in das Salz eingebunden werden kann,
wobei die Durchführung des Verfahrens mittels eines Systems erfolgt, das aus mehreren rotierenden Schaufelrädern besteht, die auf einer drehbaren zylindrischen Stange angeordnet und nicht-permanent mit dieser gekoppelt sind, welche durch eine kontinuierliche, langsame Bewegung von weniger als 10 Umdrehungen pro Minute aus erneuerbarer Energie (Wind- und Sonnenenergie) angetrieben wird,
wobei die Schaufeln in den mittleren Teil eines Meerwasserverdunstungsbeckens eingebracht und teilweise (nur wenige Millimeter unter die Oberfläche des hypersalinen Wassers) eingetaucht werden, wobei an ihren Rändern und über die gesamte Schaufelbreite ein kontinuierlicher Abtropfvorgang entsteht, der zu einer gemeinsamen Präzipitation des Salzes und der lebenden Mikroalgen-Biomasse führt,
wobei in den genannten Meersalzverdunstungsbecken von Natur aus eine hohe Konzentration der *Dunaliella salina* vorliegt und das Medium einen Kristallisationssalzgehalt von über 250 g/l aufweist.

## Revendications

1. Méthode de production d'un condiment culinaire par cristallisation conjointe du sel et de la microalgue *Dunaliella salina* dans des bassins d'évaporation de sel marin, qui permet d'intégrer la biomasse de la microalgue, à l'état cellulaire intégral, à l'intérieur du sel,
dans lequel la méthode est réalisée au moyen d'un système formé de multiples roues à aubes rotatives disposées et couplées de manière non permanente par une barre cylindrique rotative, actionnée par une énergie renouvelable, éolienne et solaire, avec un mouvement continu et lent de moins de 10 tours par minute,
où les pales sont introduites dans la partie centrale d'un bassin d'évaporation de la mer et partiellement submergées, à peine quelques millimètres dans la surface de l'eau hypersaline, formant, sur leurs bords et sur toute la largeur des pales, un égouttement continu qui donne lieu à la précipitation conjointe du sel et de la biomasse de la microalgue à l'état vivant,
dans lequel, dans ledit bassin d'évaporation du sel marin, il y a naturellement une forte concentration de *Dunaliella salina* et le milieu a atteint une salinité de cristallisation supérieure à 250 g/L.
